# EUROPÄISCHE PATENTSCHRIFT

(11) **EP 1 899 291 B1**
(45) Veröffentlichungstag und Bekanntmachung des Hinweises auf die Patenterteilung: **18.03.2009**
(21) Anmeldenummer: 06777386.1
(22) Anmeldetag: 21.06.2006
(51) Int. Cl.: C07C 209/18

(54) **VERFAHREN ZUR KONTINUIERLICHEN HERSTELLUNG EINES AMINS**
METHOD FOR THE CONTINUOUS PRODUCTION OF AN AMINE
PROCEDE DE PRODUCTION EN CONTINU D'UNE AMINE

(30) Priorität: 23.06.2005 DE 102005029095
(43) Veröffentlichungstag der Anmeldung: 19.03.2008
(73) Patentinhaber: BASF SE, 67056 Ludwigshafen (DE)
(72) Erfinder: HAESE, Frank, 63128 Dietzenbach (DE); WULFF-DÖRING, Joachim, 67227 Frankenthal (DE); KÖHLER, Ulrich, 68259 Mannheim (DE); GAA, Peter, 67551 Worms (DE); PAPE, Frank-Friedrich, 67259 Kleinniedesheim (DE); MELDER, Johann-Peter, 67459 Böhl-Iggelheim (DE); JULIUS, Manfred, 67117 Limburgerhof (DE)
(86) Internationale Anmeldenummer: PCT/EP2006/063387
(87) Internationale Veröffentlichungsnummer: WO 2006/136573

(56) Entgegenhaltungen:
- EP-A- 0 701 995
- GB-A- 1 344 574
- US-A- 3 931 298
- US-A- 3 960 962

## Beschreibung

Die vorliegenden Erfindung betrifft ein Verfahren zur kontinuierlichen Herstellung eines primären aromatischen Amins durch Umsetzung eines entsprechenden aromatischen Alkohols mit Ammoniak in Gegenwart von Wasserstoff bei einer Temperatur im Bereich von 80 bis 350°C in Gegenwart eines Heterogenkatalysators.

Die Verfahrensprodukte finden u.a. Verwendung als Zwischenprodukte bei der Herstellung von Kraftstoffadditiven (US-A-3,275,554; DE-A-21 25 039 und DE-A-36 11 230), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, lonenaustauschem, Textilhilfsmitteln, Farbstoffen, Vulkanisationsbeschleunigern und/oder Emulgatoren.

Aus der US-A-5,072,044 (Du Pont) ist die Dehydrierung von Cyclohexylaminen zu den entsprechenden aromatischen Aminen an Li-dotierten Pd-Katalysatoren bekannt. Die Umsetzung verlangt hohe Reaktionstemperaturen von 360-380°C, was die Bildung von unerwünschten Nebenprodukten fördert.

In EP-A-701 995 (BASF AG) ist ein Verfahren beschrieben zur Herstellung von aromatischen Aminen aus den entsprechenden cycloaliphatischen Aminen in Gegenwart von Wasserstoff und Ammoniak und an einem bimetallischen Palladium-/Plafin-Katalysator. Das cycloaliphatische Amin muss hierfür in einem anderen Prozess erst zur Verfügung gestellt werden.

EP-A-22 751 (Ciba-Geigy AG) beschreiben die Umsetzung von Phenolen in Gegenwart von Ammoniak und Wasserstoff zu den entsprechenden Cyclohexylaminen in Gegenwart von Edelmetallkatalysatoren mit Tonerde oder Kohle als Trägermaterial. Zur Herstellung von aromatischen Aminen wird hier nichts gelehrt.

EP-A-53 819 (BASF AG) offenbart ein Verfahren zur Herstellung von cycloaliphatischen und/oder aromatischen Aminen aus Phenolen. Das eingesetzte Katalysatorsystem enthält Ru, Rh oder Pt auf einem Aluminiumoxid-Träger.

EP-A-167 996 (BASF AG) beschreibt ein Verfahren zur Herstellung von aromatischen Aminen durch Umsetzung des entsprechenden Phenols ggf. in Gegenwart des entsprechenden Phenols ggf. in Gegenwart des entsprechenden zurückgeführten cycloaliphatischen Amins in Gegenwart von Ammoniak und Wasserstoff an einem Edelmetall-Katalysator bei Normaldruck und in 2 hintereinandergeschalteten Reaktionszonen. Bevorzugt sind Aluminiumoxid-geträgerte Katalysatoren.

US-A-3,931,298 beschreibt die reduktive Aminierung von 2,6-Dimethylphenol zur Herstellung von Xylidin.

CA Abstract No. 132:336078 (CN-B-1 087 970) betrifft die Synthese von 2,6-Dimethylanilin aus 2,6-Dimethylphenol bei 180-200 °C an einem spezifischen Pd/Al₂O₃-MgO/Al₂O₃ Katalysator.

Der vorliegenden Erfindung lag die Aufgabe zugrunde, unter Überwindung eines Nachteils oder mehrerer Nachteile des Stands der Technik, ein verbessertes wirtschaftliches Verfahren zur Herstellung eines primären aromatischen Amins aufzufinden. Insbesondere soll das Verfahren bessere Ausbeuten, Raum-Zeit-Ausbeuten (RZA) und/oder Selektivitäten ermöglichen. Der verwendete Katalysator sollte verbesserte Standzeiten und damit weniger häufige Regenerierungen ermöglichen.

[Raum-Zeit-Ausbeuten werden angegeben in ,Produktmenge / (Katalysatorvolumen • Zeit)' (kg/(I_{Kat} • h)) und/oder, Produktmenge / (Reaktorvolumen • Zeit)' (kg/(I_{Reaktor} • h)].

Die Aufgabe konnte gelöst werden, indem die Synthese in der Flüssigphase oder in der Gasphase an einem bimetallischen Palladium-/Platin-Kontakt mit ZrO₂-Träger betrieben wird und die Umsetzung in einer Monostranganlage erfolgt, wobei die Monostranganlage aus einer Hintereinanderschaltung mehrerer einzelner Rohrreaktoren besteht.

Demgemäß wurde ein Verfahren zur kontinuierlichen Herstellung eines primären aromatischen Amins durch Umsetzung eines entsprechenden aromatischen Alkohols mit Ammoniak in Gegenwart von Wasserstoff bei einer Temperatur im Bereich von 80 bis 350°C in Gegenwart eines Heterogenkatalysators, dessen katalytisch aktive Masse vor dessen Reduktion mit Wasserstoff
90 bis 99,8 Gew.-% Zirkoniumdioxid (ZrO₂),
0,1 bis 5,0 Gew.-% sauerstoffhaltige Verbindungen des Palladiums und
0,1 bis 5,0 Gew.-% sauerstoffhaltige Verbindungen des Platins
enthält, gefunden, welches dadurch gekennzeichnet ist, dass die Umsetzung in einer Monostranganlage erfolgt, wobei die Monostranganlage aus einer Hintereinanderschaltung mehrerer einzelner Rohrreaktoren besteht.

Für die Synthese in der Gasphase wird der Edukt-Alkohol gezielt, bevorzugt in einem Kreisgasstrom, verdampft und gasförmig dem Reaktor zugeführt. Das Kreisgas dient zum einen der Verdampfung des Edukt-Alkohols und zum anderen als Reaktionspartner für die Aminierung.

In der Kreisgasfahrweise werden die Ausgangsstoffe (Alkohol, Wasserstoff und Ammoniak) in einem Kreisgasstrom verdampft und gasförmig dem Reaktor zugeführt.

Die Edukte (Alkohol und Ammoniak) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Die Umsetzung erfolgt in einer Monostranganlage.

Bei einer Monostranganlage kann der Rohrreaktor, in dem die Umsetzung erfolgt, aus einer Hintereinanderschaltung mehrerer (z.B. zweier oder dreier) einzelner Rohrreaktoren bestehen. Optional ist hier vorteilhaft eine Zwischeneinspeisung von Feed (enthaltend das Edukt und/oder Ammoniak und/oder H₂) und/oder Kreisgas und/oder Reaktoraustrag aus einem nachgeschalteten Reaktor möglich.

Die Kreisgasmenge liegt bevorzugt im Bereich von 40 bis 1500 m³ (bei Betriebsdruck) / [m³ Katalysator (Schüttvolumen) • h], insbesondere im Bereich von 100 bis 700 m³ (bei Betriebsdruck) / [m³ Katalysator (Schüttvolumen) • h].

Das Kreisgas enthält bevorzugt mindestens 10, besonders 50 bis 100, ganz besonders 80 bis 100, Vol.% H₂.

Für die Synthese in der Flüssigphase sind alle Edukte und Produkte geeignet, welche schwer verdampfbar oder thermisch labil sind. In diesen Fällen kommt als weiterer Vorteil hinzu, dass auf eine Verdampfung und Rekondensation des Amins im Prozess verzichtet werden kann.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang wird das oxidische Trägermaterial Zirkoniumdioxid (ZrO₂) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper- beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:
Zirkoniumdioxid (ZrO₂), sauerstoffhaltige Verbindungen des Palladiums und sauerstoffhaltige Verbindungen des Platins.

Die Summe der o. g. Bestandteile der katalytisch aktiven Masse beträgt üblicherweise 70 bis 100 Gew.-%, bevorzugt 80 bis 100 Gew.%, besonders bevorzugt 90 bis 100 Gew.-%, besonders > 95 Gew.-%, ganz besonders > 98 Gew.-%, insbesondere > 99 Ges.-%, z.B. besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen IA bis VI A und IB bis VII B und VIII des Periodensystems, enthalten.

Beispiele für solche Elemente bzw. deren Verbindungen sind:
Übergangsmetalle, wie Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie Na₂O; Alkalimetallcarbonate; Erdalkalimetalloxide, wie SrO; Erdalkalimetallcarbanate, wie MgCO₃, CaCO₃ und Ba-CO₃; Boroxid (B₂O₃).

Bevorzugt enthält die katalytisch aktive Masse der im erfindungsgemäßen Verfahren eingesetzten Katalysatoren kein Ruthenium, kein Kupfer, kein Cobalt, kein Eisen und/oder kein Nickel.

Die katalytisch aktive Masse der im erfindungsgemäßen Verfahren eingesetzten Katalysatoren enthält nach deren letzter Wärmebehandlung und vor dessen Reduktion mit Wasserstoff
90 bis 99,8 Gew.-%, bevorzugt 98 bis 99,6 Gew.-%, besonders bevorzugt 98,8 bis 99,2 Gew.-% Zirkoniumdioxid (ZrO₂),
0,1 bis 5,0 Gew.-%, bevorzugt 0,2 bis 1,0 Gew.%, besonders bevorzugt 0,4 bis 0,6 Gew.-%, sauerstoffhaltige Verbindungen des Palladiums und
0,1 bis 5,0 Gew.-%, bevorzugt 0,2 bis 1,0 Gew.-%, besonders bevorzugt 0,4 bis 0,6 Gew.%, sauerstoffhaltige Verbindungen des Platins.

Zur Herstellung der im erfindungsgemäßen Verfahren verwendeten Katalysatoren sind verschiedene Verfahren möglich. Hier sind z.B. die bekannten Fällungsmethoden zu nennen.

Die im erfindungsgemäßen Verfahren verwendeten Katalysatoren können insbesondere durch Tränkung von Zirkoniumdioxid (ZrO₂) das beispielsweise in Form von Pulver oder Formkörpern, wie Strängen, Tabletten, Kugeln oder Ringen, vorliegt, hergestellt werden.

Das Zirkoniumdioxid wird beispielsweise in der monoklinen oder tetragonalen Form, bevorzugt in der monoklinen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional kalziniert.

Die Tränkung kann nach der sogenannten "incipient wetness"-Methode erfolgen, bei der das Zirkoniumdioxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu kalzinieren. Die mehrstufige Tränkung ist vorteilhaft besonders dann anzuwenden, wenn das Zirkoniumdioxid mit einer größeren Metallmenge beaufschlagt werden soll.

Zur Aufbringung der Metallkomponenten auf das Zirkoniumdioxid kann die Tränkung gleichzeitig mit allen Metallsalzen oder in beliebiger Reihenfolge der einzelnen Metallsalze nacheinander erfolgen.

Nach der Calcinierung, die z.B. bei einer Temperatur im Bereich von 200 bis 600 °C durchgeführt wird, wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z.B. 12 bis 20 Stunden einer Stickstoff-WasserstoffAtmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Die in EP-A-701 995 offenbarten Pd/Pt/ZrO₂-Katatysatoren werden im erfindungsgemäßen Verfahren besonders bevorzugt eingesetzt.

Das erfindungsgemäße Verfahren wird kontinuierlich durchgeführt, wobei der Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Das Aminierungsmittel Ammoniak kann bezüglich der zu aminierenden alkoholischen Hydroxylgruppe in stöchiometrischen, unter- oder überstöchiometrischen Mengen eingesetzt werden.

Ammoniak wird im allgemeinen mit einem 1,5 bis 250-fachen, bevorzugt 2 bis 100-fachen, insbesondere 2 bis 10-fachen molaren Überschuss pro Mol umzusetzender alkoholischer Hydroxylgruppe eingesetzt.

Höhere Überschüsse an Ammoniak sind möglich.

Bevorzugt wird eine Abgasmenge von 5 bis 800 Normkubikmeter/h, insbesondere 20 bis 300 Normkubikmeter/h, gefahren.

Beim Arbeiten in der Flüssigphase leitet man die Edukte (Alkohol plus Ammoniak) simultan in flüssiger Phase bei Drücken von im allgemeinen 5 bis 30 MPa (50-300 bar), bevorzugt 5 bis 25 MPa, besonders bevorzugt 15 bis 25 MPa, und Temperaturen von im allgemeinen 80 bis 350 °C, besonders 100 bis 300 °C, bevorzugt 120 bis 270 °C, besonders bevorzugt 130 bis 250 °C, insbesondere 170 bis 230 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im allgemeinen im Bereich von 0,05 bis 5, bevorzugt 0,1 bis 2, besonders bevorzugt 0,2 bis 0,6, kg Alkohol pro Liter Katalysator (Schüttvolumen) und Stunde. Gegebenenfalls kann eine Verdünnung der Edukte mit einem geeigneten Lösungsmittel, wie Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol plus Ammoniak) in einem zur Verdampfung ausreichend groß gewählten Gasstrom, bevorzugt Wasserstoff, bei Drücken von im allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung betragen im allgemeinen 80 bis 350 °C, besonders 100 bis 300 °C, bevorzugt 120 bis 290 °C, besonders bevorzugt 160 bis 280 °C. Es ist dabei sowohl eine Anströmung des Katalysatorfestbetts von oben als auch von unten möglich. Den erforderlichen Gasstrom erhält man bevorzugt durch eine Kreisgasfahrweise.

Die Katalysatorbelastung liegt im allgemeinen im Bereich von 0,01 bis 2, bevorzugt 0,05 bis 0,5, kg Alkohol pro Liter Katalysator (Schüttvolumen) und Stunde.

Der Wasserstoff wird der Reaktion im allgemeinen in einer Menge von 5 bis 400 l, bevorzugt in einer Menge von 50 bis 200 I pro Mol Alkoholkomponente zugeführt, wobei die Literangaben jeweils auf Normalbedingungen umgerechnet wurden (S.T.P.).

Sowohl beim Arbeiten in der Flüssigphase als auch beim Arbeiten in der Gasphase ist die Anwendung höherer Temperaturen und höherer Gesamtdrücke und Katalysatorbelastungen möglich. Der Druck im Reaktionsgefäß, welcher sich aus der Summe der Partialdrücke des Aminierungsmittels Ammoniak, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z.B. durch eine fraktionierende Rektifikation. Das überschüssige Aminierungsmittel und der Wasserstoff werden vorteilhaft wieder in die Reaktionszone zurückgeführt. Das gleiche gilt für die eventuell nicht vollständig umgesetzte Alkoholkomponente.

Die jeweiligen Reinprodukte können aus den Rohwaren durch Rektifikation nach den bekannten Methoden erhalten werden. Die Reinprodukte fallen als Azeotrope mit Wasser an oder können in Anlehnung an die Patentanmeldungen EP-A-1 312 599 und EP-A-1 312 600 durch eine Flüssig-flüssig-Extraktion mit konzentrierter Natronlauge entwässert werden. Diese Entwässerung kann vor oder nach der Reindestillation erfolgen. Eine destillative Entwässerung in Gegenwart eines Schleppmittels nach bekannten Methoden ist auch möglich.

Für den Fall, dass die Rohware oder das aromatische Amin in der Rohware kaum oder nicht mit Wasser mischbar sind, ist eine Entwässerung durch eine Trennung der organischen und der wässrigen Phase mit bekannten Methoden auch möglich. Gemäß der gelehrten Vorgehensweise in EP-A-1 312 599 und in EP-A-1 312 600 (beide BASF AG) können von der separierten organischen Phase in einem Schritt eine oder mehrere Leichtsiederfraktionen von dem aminhaltigen Gemisch destillativ abgetrennt werden. In einem weiteren Schritt besteht die Möglichkeit, eine oder mehrere Schwersiederfraktionen von dem aminhaltigen Gemisch destillativ abzutrennen. In einem folgenden Destillationsschritt kann aus der Mischung das im wesentlichen wasserfreie Amin als Sumpfabzug oder Seitenabzug der Kolonne in reiner Form erhalten werden, welches gegebenenfalls einer weiteren Aufreinigung oder Auftrennung unterworfen wird.

Diese Einzelschritte zur Aufreinigung des Amins können gegebenenfalls auch in einer einzigen Kolonne batchweise oder kontinuierlich durchgeführt werden, wobei eine Abtrennung der Leichtsieder über den Kopfabzug und/oder den Seitenabzug des Verstärkungsteils der Kolonne, eine Abtrennung der Schwersiederfraktionen über den Sumpfabzug der Destillationskolonne und die Abtrennung des reinen Amins über den Seitenabzug im Abtriebsteil der Kolonne erfolgen kann.

In einer besonders bevorzugten Variante kommt als kontinuierliche Destillationskolonne eine Trennwandkolonne zum Einsatz.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Durch die Verwendung von Ammoniak als Aminierungsmittel wird die alkoholische Hydroxylgruppe des eingesetzten aromatischen Alkohols unter Erhalt der Position am aromatischen Ring in die primäre Aminogruppe (-NH₂) umgewandelt.

Als aromatische Alkohole eignen sich praktisch alle Alkohole mit aromatischer OH-Funktion. D.h. die OH-Gruppe ist an ein sp²-hybridisiertes C-Atom in einem aromatischen Ring gebunden. Der aromatische Ring kann neben C-Atomen auch ein oder mehrere Heteroatome, wie N, O oder S, aufweisen. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige aromatische Alkohole aminiert werden, so hat man es über die Steuerung der Reaktionsbedingungen in der Hand, bevorzugt entsprechende Aminoalkohole oder mehrfach aminierte Produkte zu erhalten.

Bevorzugt werden beispielsweise die folgenden aromatischen Alkohole aminiert: Phenol, wobei der Phenylrest einen oder mehrere Alkylreste, insbesondere C₁₋₉-Alkylreste und C₅₋₆-Cycloalkylreste, und/oder Arylreste als Substituenten tragen kann, 1- oder 2-Naphthol, wobei der Naphthylrest einen oder mehrere Alkylreste, insbesondere C₁₋₉-Alkylreste und C₅₋₆-Cycloalkylreste, und/oder Arylreste als Substituenten tragen kann.

C₁₋₉-Alkylreste, bevorzugt C₁₋₃-Alkylreste, sind z.B. Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Nonyl.

Arylreste sind z.B. Phenyl, 1-Naphthyl, 2-Naphthyl.

Beispiele hierfür sind: ortho-, meta- und para-Kresol, ortho-Ethylphenol, ortho-n-Butylphenol, ortho-sec.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-cyclohexyl-phenol, 2,6-Diethylphenol, 2,5-Diisopropylphenol, 2-Methyl-6-sec.-butylphenol, 3-tert.-Butylphenol, 2,6-Diisopropylphenol, 2,6-Di-sec.-Butylphenol, 2,6-Dicyclohexylphenol, alpha-Naphthol, beta-Naphthol, Bisphenol A (= 2,2-Di(p-hydroxyphenyl)propan, Hydrochinon, Mono-, Di-, Tri- oder Tetraalkylhydrochinone, insbesondere mit C₁₋₉-Alkylresten (unabhängig voneinander) substituierte Hydrochinone, z.B. Monomethylhydrochinon, Tetramethylhydrochinon.

Die als Edukt eingesetzten aromatischen Alkohole, insbesondere Phenole, sind gut zugängliche Verbindungen (z.B. Houben Weyl, Methoden, Band 6/lc).

Mit dem erfindungsgemäßen Verfahren bevorzugt hergestellte aromatische Amine sind 2,6-Di-(C₁₋₉-alkyl)-aniline aus den entsprechenden 2,6-Di-(C₁₋₉-alkyl)-phenolen. Beispiele sind 2,6-Dimethylanilin (2,6-Xylidin), 2,6-Diethylanilin, 2-Methyl-6-ethylanilin, 2,6-Diisopropylanilin, 2-Isopropyl-6-methylanilin und 2-Isopropyl-6-ethylanilin.

Mit dem erfindungsgemäßen Verfahren besonders bevorzugt hergestelltes aromatische Amin ist 2,6-Dimethylanilin (2,6-Xylidin) durch Umsetzung von 2,6-Dimethyl-phenol.

Mit dem erfindungsgemäßen Verfahren, insbesondere gemäß den Ansprüchen 17 oder 18, ist insbesondere 2,6-Dimethylanilin (2,6-Xylidin) mit einer Reinheit von ≥ 99 Gew.-%, besonders ≥ 99,5 Gew.-%, ganz besonders ≥ 99,85 Gew.%, und einem Gehalt an 2,6-Dimethylphenol von ≤ 0,1 Gew.-%, besonders s 0,05 Gew.-%, ganz besonders ≤ 0,02 Gew.-%, z.B. 0 bis 0,015 Gew.%, aus 2,6-Dimethylphenol und Ammoniak herstellbar.

Die o.g. Gehalte in Gew.-% werden wie folgt gaschromatografisch bestimmt:

| | |
|---|---|
| Trennsäule | DB WAX (Polyethylenglycol) |
| Länge (m): | 30 |
| Filmdicke (µm): | 0,5 |
| Innendurchmesser (mm): | 0,25 |
| Trägergas: | Helium |
| Vordruck (bar): | 1,0 |
| Split (ml/Min.): | 100 |
| Septumspülung (ml/Min.): | 4 |
| Ofentemperatur (°C): | 80 |
| Vorheizzeit (Min.): | 3 |
| Rate (°C/Min.): | 5 |
| Ofentemperatur (°C): | 240 |
| Nachheizzeit (Min.): | 30 |
| Injektortemperatur (°C): | 250 |
| Detektortemperatur (°C): | 260 |
| Injektion: | HP 7673-Autosampler |
| Injetionsvolumen (mikrol): | 0,2 |
| Detektorlyp: | FID |
| GC-Methode: | GC-FI.% - Methode |

### Beschreibung der Probenvorbereitung:

Die Probe wird ggf. 4 h bei 60°C aufgeschmolzen (2,6-Dimethylphenol schmilzt bei etwa 45°C). Ca. 1 g der aufgeschmolzenen Probe werden in 50 ml Dichlormethan gelöst und analysiert.

Besonders bevorzugt weist das im erfindungsgemäßen Verfahren zur Herstellung von 2,6-Dimethylanilin eingesetzte 2,6-Dimethylphenol folgende Spezifikation auf:

| | |
|---|---|
| Assay | Min. 99,5 Gew-% |
| Cresols (all isomers) | Max. 1500 ppm |
| Xylenoles (all iso mers) | Max. 1000 ppm |
| Anisol | Max. 1000 ppm |
| Phenol | Max. 100 ppm |
| Moisture | Max. 0,5 Gew-% |
| Sulphur | Max. 1 ppm |
| Chlorine | Max. 1 ppm |
| Bromine | Max. 1 ppm |

| | |
|---|---|
| ppm-Angaben bezichen sich auf das Gewicht. | |

Die Bestimmung von Wasser erfolgt mittels Karl-Fischer- Titration.

### Bestimmung von Schwefel:

Coulometrische Bestimmung über Verbrennung
Gerät: Fa. Euroglas (LHG), Typ ECS 1200; Literatur: DIN 51400 Teil 7

### Bestimmung von Halogen:

Coulometrische Bestimmung über Verbrennung
Gerät: Fa. Euroglas (LHG), Typ ECS 1200
Literatur: F. Ehrcnberger "Quantitative organische Elementaranalyse"
   ISBN 3-527-28056-1
   DIN 51408 Teil 2, "Bestimmung des Chlorgehaltes

### Beispiele

Für alle Beispiele wurde der bimetallische Palladium-/Platin-Katalysator gemäß Beispiel 4 (Seite 6, Zeilen 12-15) von EP-B1-701 995 verwendet und auch nach der dort beschriebenen Methode (Seite 4, Zeilen 47-52) aktiviert. Danach wurde der geträgerte Edelmetall-Katalysator in den Reaktor eingebaut und anschließend in einem Stickstoff/Wasserstoffstrom drucklos oder unter Betriebsdruck bei 200°C reduziert.

### Herstellung von 2,6-Dimethylanilin

in zwei hintereinander geschalteten Reaktoren mit jeweils 10 Liter Katalysatorfüllung wurde bei 2 bar Gesamtdruck ein Kreisgasstrom aus 170 kg/h Ammoniak und 20 kg/h Wasserstoff eingestellt. In diesen Strom wurden kontinuierlich 122 kg/h 2,6-Dimethylphenol gegeben und verdampft. Die gasförmige Mischung wurde bei 200 bis 220°C über das Katalysatorbett des ersten Reaktors und bei 230 bis 270°C über das Katalysatorbett des zweiten Reaktors geströmt. Die Ausbeute an 2,6-Xylidin nach dem zweiten Reaktor betrug 95 %.

## Patentansprüche

1. Verfahren zur kontinuierlichen Herstellung eines primären aromatischen Amins durch Umsetzung eines entsprechenden aromatischen Alkohols mit Ammoniak in Gegenwart von Wasserstoff bei einer Temperatur im Bereich von 80 bis 350 °C in Gegenwart eines Heterogenkatalysators, dessen katalytisch aktive Masse vor dessen Reduktion mit Wasserstoff
90 bis 99,8 Gew.-% Zirkoniumdioxid (ZrO₂),
0,1 bis 5,0 Gew.-% sauerstoffhaltige Verbindungen des Palladiums und
0,1 bis 5,0 Gew.-% sauerstoffhaltige Verbindungen des Platins enthält, **dadurch gekennzeichnet, dass** die Umsetzung in einer Monostranganlage erfolgt, wobei die Monostranganlage aus einer Hintereinanderschaltung mehrerer einzelner Rohrreaktoren besteht.

2. Verfahren zur kontinuierlichen Herstellung eines primären aromatischen Amins durch Umsetzung eines entsprechenden aromatischen Alkohols mit Ammoniak in Gegenwart von Wasserstoff bei einer Temperatur im Bereich von 80 bis 350 °C in Gegenwart eines Heterogenkatalysators, dessen katalytisch aktive Masse vor dessen Reduktion mit Wasserstoff
90 bis 99,8 Gew.-% Zirkoniumdioxid (ZrO₂),
0,1 bis 5,0 Gew.-% sauerstoffhaltige Verbindungen des Palladiums und
0,1 bis 5,0 Gew.-% sauerstoffhaltige Verbindungen des Platins enthält, **dadurch gekennzeichnet, dass** die Umsetzung in einer Monostranganlage erfolgt, wobei die Monostranganlage aus einer Hintereinanderschaltung zweier oder dreier einzelner Rohrreaktoren besteht.

3. Verfahren nach einem der vorhergehenden Ansprüche, **dadurch gekennzeichnet, dass** man die Umsetzung bei einer Temperatur im Bereich von 120 bis 300 °C durchführt.

4. Verfahren nach einem der vorhergehenden Ansprüche, **dadurch gekennzeichnet, dass** man die Umsetzung in der Flüssigphase bei einem Absolutdruck im Bereich von 5 bis 30 MPa oder in der Gasphase bei einem Absolutdruck im Bereich von 0,1 bis 40 MPa durchführt.

5. Verfahren nach einem der vorhergehenden Ansprüche, **dadurch gekennzeichnet, dass** die katalytisch aktive Masse des Katalysators vor dessen Reduktion mit Wasserstoff
98 bis 99,6 Gew.-% Zirkoniumdioxid (ZrO₂),
0,2 bis 1,0 Gew.-% sauerstoffhaltige Verbindungen des Palladiums und
0,2 bis 1,0 Gew.-% sauerstoffhaltige Verbindungen des Platins enthält.

6. Verfahren nach einem der Ansprüche 1 bis 4, **dadurch gekennzeichnet, dass** die katalytisch aktive Masse des Katalysators vor dessen, Reduktion mit Wasserstoff
98,8 bis 99,2 Gew.-% Zirkoniumdioxid (ZrO₂),
0,4 bis 0,6 Gew.-% sauerstoffhaltige Verbindungen des Palladiums und
0,4 bis 0,6 Gew.-% sauerstoffhaltige Verbindungen des Platins enthält.

7. Verfahren nach einem der vorhergehenden Ansprüche, **dadurch gekennzeichnet, dass** der Ammoniak in der 1,5- bis 250-fachen molaren Menge bezogen auf den eingesetzten aromatischen Alkohol eingesetzt wird.

8. Verfahren nach einem der Ansprüche 1 bis 6, **dadurch gekennzeichnet, dass** der Ammoniak in der 2,0- bis 10-fachen molaren Menge bezogen auf den eingesetzten aromatischen Alkohol eingesetzt wird.

9. Verfahren nach einem der vorhergehenden Ansprüche, **dadurch gekennzeichnet, dass** der Katalysator im Reaktor als Festbett angeordnet ist.

10. Verfahren nach einem der vorhergehenden Ansprüche, **dadurch gekennzeichnet, dass** die Umsetzung bei einer Kreisgasfahrweise erfolgt.

11. Verfahren nach einem der vorhergehenden Ansprüche, **dadurch gekennzeichnet, dass** man den Alkohol als wässrige Lösung einsetzt.

12. Verfahren nach einem der vorhergehenden Ansprüche, **dadurch gekennzeichnet, dass** man den Ammoniak als wässrige Lösung einsetzt.

13. Verfahren nach einem der vorhergehenden Ansprüche zur Herstellung eines Phenylamins, wobei der Phenylrest einen oder mehrere C₁₋₉-Alkylreste als Substituenten tragen kann.

14. Verfahren nach einem der Ansprüche 1 bis 12 zur Herstellung von Anilin durch Umsetzung von Phenol mit Ammoniak.

15. Verfahren nach einem der Ansprüche 1 bis 12 zur Herstellung eines 2,6-Di-(C₁₋₉-alkyl)-anilins.

16. Verfahren nach einem der Ansprüche 1 bis 12 zur Herstellung von 2,6-Dimethylanilin (2,6-Xylidin) durch Umsetzung von 2,6-Dimethylphenol mit Ammoniak.

17. Verfahren nach einem der vorhergehenden Ansprüche, **dadurch gekennzeichnet, dass** man aus dem Reaktionsrohprodukt die organische Phase abtrennt und diese nachfolgend in einer Destillationskolonne kontinuierlich destillativ auftrennt, wobei das primäre aromatische Amin über einen seitlichen Abzug im Abtriebsteil der Kolonne, Leichtersieder und Wasser über Kopf und Schwerersieder über Sumpf abgezogen werden.

18. Verfahren nach dem vorhergehenden Anspruch, **dadurch gekennzeichnet, dass** es sich bei der Destillationskolonne um eine Trennwandkolonne handelt.

## Claims

1. A process for the continuous preparation of a primary aromatic amine by reaction of a corresponding aromatic alcohol with ammonia in the presence of hydrogen at a temperature in the range from 80 to 350°C in the presence of a heterogeneous catalyst whose catalytically active composition before reduction with hydrogen comprises
from 90 to 99.8% by weight of zirconium dioxide (ZrO₂),
from 0.1 to 5.0% by weight of oxygen-comprising compounds of palladium and
from 0.1 to 5.0% by weight of oxygen-comprising compounds of platinum,
wherein the reaction is carried out in a single-stream plant comprising a plurality of individual tube reactors connected in series.

2. A process for the continuous preparation of a primary aromatic amine by reaction of a corresponding aromatic alcohol with ammonia in the presence of hydrogen at a temperature in the range from 80 to 350°C in the presence of a heterogeneous catalyst whose catalytically active composition before reduction with hydrogen comprises
from 90 to 99.8% by weight of zirconium dioxide (ZrO₂),
from 0.1 to 5.0% by weight of oxygen-comprising compounds of palladium and
from 0.1 to 5.0% by weight of oxygen-comprising compounds of platinum,
wherein the reaction is carried out in a single-stream plant comprising two or three individual tube reactors connected in series.

3. The process according to either of the preceding claims, wherein the reaction is carried out at a temperature in the range from 120 to 300°C.

4. The process according to any of the preceding claims, wherein the reaction is carried out in the liquid phase at an absolute pressure in the range from 5 to 30 MPa or in the gas phase at an absolute pressure in the range from 0.1 to 40 MPa.

5. The process according to any of the preceding claims, wherein the catalytically active composition of the catalyst before reduction with hydrogen comprises
from 98 to 99.6% by weight of zirconium dioxide (ZrO₂),
from 0.2 to 1.0% by weight of oxygen-comprising compounds of palladium and
from 0.2 to 1.0% by weight of oxygen-comprising compounds of platinum.

6. The process according to any of claims 1 to 4, wherein the catalytically active composition of the catalyst before reduction with hydrogen comprises
from 98.8 to 99.2% by weight of zirconium dioxide (ZrO₂),
from 0.4 to 0.6% by weight of oxygen-comprising compounds of palladium and
from 0.4 to 0.6% by weight of oxygen-comprising compounds of platinum.

7. The process according to any of the preceding claims, wherein the ammonia is used in a from 1.5-to 250-fold molar amount based on the aromatic alcohol used.

8. The process according to any of claims 1 to 6, wherein the ammonia is used in a from 2.0- to 10-fold molar amount based on the aromatic alcohol used.

9. The process according to any of the preceding claims, wherein the catalyst is arranged as a fixed bed in the reactor.

10. The process according to any of the preceding claims, wherein the reaction is carried out in a gas recycle mode.

11. The process according to any of the preceding claims, wherein the alcohol is used as aqueous solution.

12. The process according to any of the preceding claims, wherein the ammonia is used as aqueous solution.

13. The process according to any of the preceding claims for the preparation of a phenylamine, where the phenyl radical may bear one or more C₁₋₉-alkyl radicals as substituents.

14. The process according to any of claims 1 to 12 for the preparation of aniline by reaction of phenol with ammonia.

15. The process according to any of claims 1 to 12 for the preparation of a 2,6-di(C₁₋₉-alkyl) aniline.

16. The process according to any of claims 1 to 12 for the preparation of 2,6-dimethylaniline (2,6-xylidine) by reaction of 2,6-dimethylphenol with ammonia.

17. The process according to any of the preceding claims, wherein the organic phase is separated off from the crude reaction product and is subsequently worked up continuously by distillation in a distillation column, with the primary aromatic amine being taken off via a side offtake in the stripping section of the column, low boilers and water being taken off at the top and high boilers being taken off at the bottom.

18. The process according to the preceding claim, wherein the distillation column is a dividing wall column.

## Revendications

1. Procédé de fabrication continue d'une amine primaire aromatique par réaction d'un alcool aromatique correspondant avec de l'ammoniac en présence d'hydrogène à une température de 80 à 350 °C en présence d'un catalyseur hétérogène, dont la masse catalytiquement active contient avant sa réduction avec l'hydrogène
90 à 99,8 % en poids de dioxyde de zirconium (ZrO₂),
0,1 à 5,0 % en poids de composés du palladium contenant de l'oxygène et
0,1 à 5,0 % en poids de composés du platine contenant de l'oxygène,
**caractérisé en ce que** la réaction a lieu dans une unité à gaine unique, l'unité à gaine unique étant constituée d'une connexion successive de plusieurs réacteurs tubulaires individuels.

2. Procédé de fabrication continue d'une amine primaire aromatique par réaction d'un alcool aromatique correspondant avec de l'ammoniac en présence d'hydrogène à une température de 80 à 350 °C en présence d'un catalyseur hétérogène, dont la masse catalytiquement active contient avant sa réduction avec l'hydrogène
90 à 99,8 % en poids de dioxyde de zirconium (ZrO₂),
0,1 à 5,0 % en poids de composés du palladium contenant de l'oxygène et
de l'oxygène,
**caractérisé en ce que** la réaction a lieu dans une unité à gaine unique, l'unité à gaine unique étant constituée d'une connexion successive de deux ou trois réacteurs tubulaires individuels.

3. Procédé selon l'une quelconque des revendications précédentes, **caractérisé en ce que** la réaction est réalisée à une température de 120 à 300°C.

4. Procédé selon l'une quelconque des revendications précédentes, **caractérisé en ce que** la réaction est réalisée en phase liquide à une pression absolue de 5 à 30 MPa ou en phase gazeuse à une pression absolue de 0,1 à 40 MPa.

5. Procédé selon l'une quelconque des revendications précédentes, **caractérisé en ce que** la masse catalytiquement active du catalyseur contient avant sa réduction avec l'hydrogène
98 à 99,6 % en poids de dioxyde de zirconium (ZrO₂),
0,2 à 1,0 % en poids de composés du palladium contenant de l'oxygène et
0,2 à 1,0 % en poids de Composés du platine contenant de l'oxygène.

6. Procédé selon l'une quelconque des revendications 1 à 4, **caractérisé en ce que** la masse catalytiquement active du catalyseur contient avant sa réduction avec l'hydrogène
98,8 à 99,2 % en poids de dioxyde de zirconium (ZrO₂),
0,4 à 0,6 % en poids de composés du palladium contenant de l'oxygène et
0,4 à 0,6% en poids de composés du platine contenant de l'oxygène.

7. Procédé selon l'une quelconque des revendications précédentes, **caractérisé en ce que** l'ammoniac est utilisé en une quantité molaire de 1,5 à 250 fois par rapport à l'alcool aromatique utilisé.

8. Procédé selon l'une quelconque des revendications 1 à 6, **caractérisé en ce que** l'ammoniac est utilisé en une quantité molaire de 2,0 à 10 fois par rapport à l'alcool aromatique utilisé.

9. Procédé selon l'une quelconque des revendications précédentes, **caractérisé en ce que** le catalyseur est disposé dans le réacteur sous la forme d'un lit solide.

10. Procédé selon l'une quelconque des revendications précédentes, **caractérisé en ce que** la réaction a lieu en circulation gazeuse.

11. Procédé selon l'une quelconque des revendications précédentes, **caractérisé en ce que** l'alcool est utilisé sous la forme d'une solution aqueuse.

12. Procédé selon l'une quelconque des revendications précédentes, **caractérisé en ce que** l'ammoniac est utilisé sous la forme d'une solution aqueuse.

13. Procédé selon l'une quelconque des revendications précédentes pour la fabrication d'une phénylamine, dans lequel le radical phényle peut porter un ou plusieurs radicaux alkyle en C₁₋₉ en tant que substituants.

14. Procédé selon l'une quelconque des revendications 1 à 12 pour la fabrication d'aniline par réaction de phénol avec de l'ammoniac.

15. Procédé selon l'une quelconque des revendications 1 à 12 pour la fabrication d'une 2,6-di-(C₁₋₉-alkyl)-aniline.

16. Procédé selon l'une quelconque des revendications 1 à 12 pour la fabrication d'une 2,6-diméthylaniline (2,6-xylidine) par réaction de 2,6-diméthylphénol avec de l'ammoniac.

17. Procédé selon l'une quelconque des revendications précédentes, **caractérisé en ce que** la phase organique est séparée du produit brut de la réaction et celle-ci est séparée ultérieurement par distillation continue dans une colonne de distillation, l'amine primaire aromatique étant extraite par une sortie latérale dans la zone de rectification de la colonne, les composés de faible point d'ébullition et l'eau au niveau de la tête et les composés de point d'ébullition élevé au niveau du fond.

18. Procédé selon l'une quelconque des revendications précédentes, **caractérisé en ce que** la colonne de distillation est une colonne de partage.
